(19) Europäisches Patentamt / European Patent Office / Office européen des brevets

(11) **EP 2 185 717 B1**

(12) **EUROPEAN PATENT SPECIFICATION**

(45) Date of publication and mention
of the grant of the patent:
**14.12.2011 Bulletin 2011/50**

(51) Int Cl.:
**C12P 19/44** (2006.01)    **A23L 1/185** (2006.01)

(21) Application number: **08775209.3**

(22) Date of filing: **18.07.2008**

(86) International application number:
**PCT/EP2008/059433**

(87) International publication number:
**WO 2009/016049 (05.02.2009 Gazette 2009/06)**

(54) **Production of maltobionate**

Herstellung von Maltobionat

Procédé de production du maltobionate

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **27.07.2007 EP 07113338**

(43) Date of publication of application:
**19.05.2010 Bulletin 2010/20**

(73) Proprietor: **Novozymes A/S
2880 Bagsværd (DK)**

(72) Inventor: **NIELSEN, Per Munk
DK-3400 Hilleroed (DK)**

(56) References cited:
**WO-A-2007/101846**

• XU ET AL: "A novel carbohydrate:acceptor
oxidoreductase from Microdochium nivale"
EUROPEAN JOURNAL OF BIOCHEMISTRY, vol.
268, 2001, pages 1136-1142, XP002500983
• GREEN: "After 30 years ... the future of
hydroxyacids" JOURNAL OF COSMETIC
DERMATOLOGY, vol. 4, 2005, pages 44-45,
XP002500984
• FAN ET AL: "Characterization of kinetics and
thermostability of Acremonium strictum
glucooligosaccharide oxidase"
BIOTECHNOLOGY AND BIOENGINEERING, vol.
68, 2000, pages 231-237, XP002500985
• VIDGREN ET AL: "Characterization and
functional analysis of the MAL and MPH loci for
maltose utilization in some ale and lager yeast
strains", APPLIED AND ENVIRONMENTAL
MICROBIOLOGY, 2005, pages 7846-7857,
XP002419699,

Description

FIELD OF THE INVENTION

The invention provides a way of preserving beer products with maltobionate produced directly from maltose already present in the beer product, using an enzymatic process.

BACKGROUND OF THE INVENTION

Prevention oh oxidative degradation of food and feed products is very important for the preservation of the quality of the products. Oxidation processes in the products can lead to changes in colour, flavour, aroma or other organoleptic unacceptable changes. Furthermore, oxidation may cause damage to essential amino acids and result in the loss of vitamins. In particular, food products containing polyunsaturated fatty acids are susceptible to oxidation, potentially resulting in rancid food products.

An oxidation reaction occurs when a food molecule, e.g. a fatty acid, combines with oxygen in the presence of free radicals; trace metals, such as Fe and Cu; or reactive oxygen species, such as singlet oxygen, peroxides or hydroperoxide. Antioxidants are used to suppress these reactions Examples of generally utilized antioxidants are butylhydroxyanisole (BHA) and butylhydroxytoluene (BHT), which are mostly used in foods that are high in fats and oils, as well as sulfites, which are used primarily as antioxidants to prevent or reduce discoloration of fruits and vegetables. However, BHA and BHT are suspected of causing tumors when used in high concentrations and may therefore not be safe for human health and sulfites are known to destroy vitamin B. For these reasons, biological or natural antioxidants, such as, tocopherol (Vitamin E), L-ascorbic acid, citric acid, melanoidin, flavonoids and gallic acid are generally preferred. Chelating agents such as EDTA, siderohores (iron chelating agents from microorganisms), citric acid and lactobionic acid have also been used to address problems with oxidation due to their ability to prevent trace metals from provoking oxidation.

US Patent No. 3,899,604 discloses the production of maltobionic acid from maltose by fermentatative oxidation using a *Pseudomonas gravlolens* species and the use of maltobionic acid as a food additive; maltobionic acid has a mildly sour flavour and also contributes to the viscosity of the food products in which it is contained. Futhermore, maltobionic acid may enhance the natural smell and taste of certain food products (flavour improver) as described in US Patent No. 3,829,583. There is, however, no indication that maltobionate contributes with an antioxidative effect in the food.

European Patent No. 0 384 534 B1 discloses the production of maltobionic acid from maltose by fermentatative oxidation using a *Pseudomonas cepacia* strain.

Maltose and maltotriose are the major sugars in brewer's wort (App Env Microbiol, 2005, pp. 7846-7857).

Oxidation is not only an issue during prolonged storage, but can also cause undesirable changes to a product during production, in particular when oxygen is present during production. Consequently, methods for providing natural antioxidants during the production of food would be desirable.

SUMMARY OF THE INVENTION

The present invention provides a method for impeding oxidation reactions in beer products by production of maltobionate from maltose present in the wort or mash by an enzymatic process.

DESCRIPTION OF THE INVENTION

According to the present invention, oxidative reactions in beer products can be prevented or impeded during their manufacture by maltobionate. To our knowledge, it is the first time maltobionate has been used as an antioxidant in beer during and after production.

*Definitions:*

The term "adjunct" is understood as the part of the grist which is not barley malt. The adjunct may comprise any starch-rich plant material, e.g. uhmalted grain, such as barley, rice, corn. wheat, rye, sorghum and readily fermentable sugar and/or syrup.

As used herein the term "**a fraction isolated from a food or feed production process**" is to be understood as an isolated portion which essentially contains all the ingredients normally. used in the part of the process where it is isolated from. Preferably, the fraction contains an increased amount of starch compared to what is normally present in the part of the process from which it is isolated. The fraction can be obtained at any step during the production process, and can also be the final product. In the event that an increased amount of starch is desired an additional amount of the starch containing ingredient of the process or pure starch can be added to the isolated fraction. process, and can also be the final product. In the event that an increased amount of starch is desired an additional amount of the starch containing ingredient of the process or pure starch can be added to the isolated fraction.

As used herein the term "**grist**" is understood as the starch or sugar containing material that is the basis for beer production, e.g. the barley malt and the adjunct.

The term "**isolated enzyme**" as used herein refers to a polypeptide with the described enzymatic activity, where the polypeptide is at least 20% pure, preferably at least 40% pure, more preferably at least 60% pure, even more preferably at least 80% pure, most preferably at least 90% pure, and even most preferably at least 95% pure, as determined by SDS-PAGE.

The term "**malt**" is understood as any malted cereal grain, in particular barley.

The term "**maltobionate**" as used herein relates to maltobionic acid (CAS Reg. No. 534-42-9; 4-O-alpha-D-Glucopyranosyl-D-gluconic acid) or salts thereof. Suitable salts include, but are not limited to, Na-maltobionate, Ca-maltobionate, $NH_4$-maltobionate and K-maltobionate.

The term "**mash**" is understood as a starch containing slurry comprising grist steeped in water.

The term "**pure maltose**" is to be understood as a composition which only contains maltose, water, inorganic salts and potentially a buffering agent such as inorganic salts (e.g. phosphate salt, carbonate salt, hydroxide salt, etc.), organic salts (e.g. citrate phosphate, sodium acetate, etc.), and other organic buffers (e.g. HEPES, Tris, etc.).

The term "**weak**" vs "**strong**" base refers to the ability of the base to dissociate. In the present context, a weak base is defined as a base having a pKb-value of at least 3.5 (for diprotic bases like $CO_3^{2-}$ this pKb-value refers to the first step).

The term "**wort**" is understood as the unfermented liquor run-off following extracting the grist during mashing.

### *Enzymes*

Maltobionate can be generated by oxidation of maltose. The oxidation can be performed using bromide, this is however not desirable in a food production process.

In the present invention, the conversion of maltose to maltobionate is the product of an enzymatic reaction where an oxidoreductase which has substrate specificity for maltose, catalyzes the conversion. Oxidoreductases are enzymes that catalyze the transfer of electrons from one molecule to another. Dehydrogenases and oxidases belong to the enzyme class of oxidoreductases. Generally, dehydrogenases need the presence of a cofactor, e.g. NAD/NADP or a flavin coenzyme, such as, FAD or FMN, and this may also be the case for oxidases. Unless anything else is suggested, the enzymes described below and throughout the description are isolated enzymes with co-factor if required.

One category of oxidoreductases, suitable for use in the present invention, is oxidases that catalyze an oxidation/reduction reaction involving molecular oxygen ($O_2$) as the electron acceptor. In these reactions, oxygen is reduced to water ($H_2O$) or hydrogen peroxide ($H_2O_2$). In particular, carbohydrate oxidases that catalyse the conversion of maltose to maltose-delta-lactone that immediately decomposes in water to form maltobionate. The process generates hydrogen peroxide. The net reaction scheme may be described as:

$$\text{maltose} + O_2 + H_2O \rightarrow \text{maltobionate} + H_2O_2 \qquad \text{(equation 1)}$$

A number of suitable carbohydrate oxidases capable of converting maltose to maltobionate, are known and available to the skilled person. Examples of such carbohydrate oxidases are aldose oxidase, cellobiose oxidase (EC 1.1.99.18), pyranose oxidase (EC1.1.3.10), and hexose oxidase (EC1.1.3.5). By studying EC 1.1.3._, EC 1.2.3._, EC 1.4.3._, and EC 1.5.3 _or similar enzyme classes based on the recommendations of the Nomenclature Committee of the International Union of Biochemistry and Molecular Biology (IUBMB), other examples of useful carbohydrate oxidases are easily recognized by one skilled in the art.

A preferred carbohydrate oxidase is a microbial carbohydrate oxidase, in particular an isolated carbohydrate oxidase.

Hexose oxidase (EC1.1.3.5) is a carbohydrate oxidase capable of oxidizing several saccharides including glucose, galactose, maltose, cellobiose and lactose. Enzymes belonging to the class of hexose oxidases are preferred enzymes in the present invention. Hexose oxidases are produced naturally by several marine algal species. Such species are i.e. found in the family *Gigartinaceae* which belong to the order *Gigartinales.* Examples of hexose oxidase producing algal species belonging to *Gigartinaceae* are *Chondrus crispus* and *Iridophycus flacci*. Also algal species of the order *Cryptomeniales* including the species *Euthora cristata* are potential sources of the hexose oxidase suitable for use in the present invention. In particular, Hexose oxidases suitable for use in the present invention are for example extracted from the red alga *Iridophycus flaccidum* (Bean and Hassid, 1956, J Biol Chem 218: 425-436) or extracted from *Chondrus crispus,* or *Euthora cristata* as described in WO96/40935, which further describes cloning and recombinant expression of hexose oxidase from *Chondrus crispus* shown as SEQ ID NOS 30 and 31 in WO96/40935. Cellobiose oxidase (EC 1.1.99.18) is a carbohydrate oxidase capable of oxidizing several saccharides including cellobiose, soluble cellooligosaccharides, lactose, xylobiose and maltose. Enzymes belonging to the class of cellobiose oxidases are also preferred enzymes in the present invention. Cellobiose oxidase is an extracellular enzyme produced by various wood-degrading fungi, such as the white-rot fungus *Phanerochaete Chrysosporium,* brown-rot fungus *Coniophora Puteana* and soft-rot fungi such as *Monilia sp., Chaetomium, cellulolyticum, Myceliophthora (Sporotrichum) thermophila, Sclerotium rolfsii and Humicola insolens* (Schou et al., 1998, Biochemical Journal 330: 565-571).

Other suitable carbohydrate oxidases can be derived, e.g. from a mitosporic *Pyrenomycetes* such as *Acremonium,* in particular, *A. strictum* deposited under ATCC 34717 or *A. strictum* T1 (Lin et al., 1991, Biochimica et Biophysica Acta 1118: 41-47); *A. fusidioides* deposited under IFO 6813; or *A. potronii* deposited under IFO 31197. In a preferred embodiment, the carbohydrate oxidase is obtained from the source disclosed in (Lin et al., 1991, Biochimica et Biophysica Acta 1118: 41-47) as well as in JP5084074.

In another preferred embodiment the carbohydrate oxidase is a carbohydrate oxidase obtained from a fungus belonging to the genus *Microdochium,* more preferably wherein the fungus is *Microdochium nivale* and even more preferably wherein the fungus is the *Microdochium nivale* deposited under CBS 100236. The oxidase isolated from CBS 100236 is described in details in WO 99/31990.

Generation of maltobionate by fermentation of bacteria of the genus *Pseudomonas* growing on a substrate containing maltose has previously been described (US 2,496,297, US 3,862,005, US 3,899,604 and EP384534). It is also possible to use dehydrogenases with the present invention. Such dehydrogenase enzyme systems may be isolated from *Psedomonas,* in particular from *P. ovalis, P. schuylkilliensis, P. graveolens* (e.g. deposited under IFO 3460), *P. fragi, P. iodinum, P. amyloderamosa* (e.g. deposited under ATCC 21262) or *P. cepacia* (e.g. deposited under CBS 659.88 or CBS 658.88).

The amount of oxidase/dehydogenase to be used will generally depend on the specific requirements and on the specific enzyme. The amount of oxidase addition preferably is sufficient to generate the desired degree of conversion of maltose to maltobionate within a specified time. Typically, an oxidase addition in the range from about 1 to about 10000 OXU per kg of substrate is sufficient, particularly from about 5 to about 5000 OXU per kg of substrate, and more particularly from about 5 to about 500 OXU per kg of substrate. It is within the general knowledge of the skilled person to adjust the amount of specific enzyme needed for conversion of maltose to maltobionate.

In the literature an Oxidase Unit (OXU) is normally defined as the amount of enzyme that oxidizes one $\mu$mol maltose per minute under specific conditions. However, in the examples provide herein OXU is defined as one mg of pure oxidase enzyme - as measured relative to an enzyme standard.

In a further aspect of the present invention, the maltobionate is produced by a two enzyme catalyzed reaction. The first reaction form maltose from the starch components present during the food or feed production, using an amylase enzyme. The second reaction is oxidizing maltose to maltobionate as described in the aspect above. The two reactions can be made simultaneously or sequentially. In a preferred embodiment the amylase reaction is made first followed by the maltose to maltobionate reaction.

Amylase is capable of hydrolyzing starch to form oligosaccharides as a main product, in particular maltose, a process which is well known to the skilled person. The amylase may be derived from a bacterium or a fungus, in particular from a strain of *Aspergillus,* preferably a strain of *A. niger* or *A. oryzae,* or from a strain of *Bacillus.* Some examples are alpha-amylase, e.g. from *Bacillus amyloliquefaciens,* and amyloglucosidase, e.g. from *A. niger.* Commercial products include BAN and AMG (products of Novo Nordisk A/S, Denmark), Grindamyl A 1000 or A 5000 (available from Grindsted Products, Denmark) and Amylase H and Amylase P (products of Gist-Brocades, The Netherlands). Beta-amylase, or other starch degrading enzymes resulting in the formation of maltose, can likewise be used.

In a further aspect of the invention catalase (EC 1.11.1.6) is added to prevent limitation of the reaction driven by the carbohydrate oxidase and to eliminate unwanted $H_2O_2$ in the end-product. A catalase is an enzyme that catalyses the reaction: $2 H_2O_2 \rightarrow O_2 + 2 H_2O$ (equation 2).

As described above carbohydrate oxidase is dependent on oxygen, but produces hydrogen peroxide. The advantage of adding catalase to the process of the present invention is that the carbohydrate oxidase is provided with oxygen and at the same time is the hydrogen peroxide which has strong oxidizing properties removed. This is particular relevant if the maltobionate is produced as an integrated part of the food or feed production process.

A number of suitable catalases are known to the skilled person, for instance, the commercially available catalase, Catazyme® from Novozymes A/S.

### *Production*

The production of maltobionate by fermentation e.g. using bacteria of the genus *Pseudomonas* growing on a substrate containing maltose is generally known in the art (US 2,496,297, US 3,862,005, US 3,899,604 and EP384534). Additionally, WO 99/31990 describes the oxidation of pure maltose to maltobionate using carbohydrate oxidase.

One aspect of the invention relates to the production of maltobionate, by processing maltose naturally present in beer production . The process for producing maltobionate comprises the following steps:

a) obtaining a maltose containing substrate applicable in a wort or mash production process;

b) converting the maltose to maltobionate by an enzymatic reaction, wherein the maltobionate is formed during a sub-process in a beer production . The enzymatic reaction in step b) is catalysed by an oxidoreductase/dehydrogenase, preferably by one of the carbohydrate oxidases described in the above, even more preferred by one of the hexose oxidases described above and most preferred by the carbohydrate oxidase obtainable from *Microdochium nivale* deposited under CBS 100236. The described process results in an almost complete conversion of maltose to maltobionate, preferably 80%, more preferably 85%, more preferably 90 %, even more preferred 95%, most preferred 99%, and even most preferred 100% maltose in the substrate is converted to maltobionate.

An advantage of this process is the components used are those which are used in beer production anyway, hence the maltobionate is not derived form any additives. Preferably, the maltose containing fraction comprise between 5% and 60%, more preferably between 10% and 40%, even more preferably between 15% and 30%, even more preferably between 20% and 25% maltose. An additional advantage of the process of the described process is that the other components from the beer product are not complicating the process.

The process complications from other components can for instance be the formation of foam as the carbohydrate oxidase reaction may require the addition of oxygen to the reaction mixture, which can create foam in protein containing reaction mixtures.

The substrate which comprises maltose is obtained from beer brewing,

In the beer brewing process maltose is naturally present, since the mashing process produces maltose for the fermentation. In particular the wort has high maltose content.

The process for the production of maltobionate must be performed under conditions allowing the carbohydrate oxidase to convert maltose to maltobionate. Such conditions include, but are not limited to, temperature, pH, oxygen, amount and characteristics of carbohydrate oxidase, other additives such as e.g. catalase and reaction/incubation time.

A suitable incubation time should allow the degree of conversion of maltose to maltobionate of interest. Generally, a suitable incubation time is selected in the range from ½ hour to 3 days, preferably, from 2 hours to 48 hours, more preferably from 5 hour to 24 hours, most preferably from 8 hours to 18 hours.

Oxygen is an important factor as the conversion of maltose to maltobionate consumes oxygen (see equation 1 above). Accordingly, if the oxygen is monitored during the enzymatic reaction one will generally observe an initial drop in the oxygen amount, which, if e.g. air is constantly provided, will return to around the initial level, when the enzyme reaction terminates. When oxygen returns to more than 90% of the initial level the enzymatic reaction has ended or at least been significantly slowed down, indicating that all the substrate (e.g. starch, dextrin and/or maltose) has been processed to maltobionate. Accordingly, a suitable incubation time could preferably be a time that at least lasts until the oxygen level in the production batch has returned to more than 90% of the initial level, especially if a maximum conversion of maltose is desired. Alternatively, the reaction can be monitored by the amount of base required to keep the pH constant. When the amount of base needed to maintain pH decreases it is an indication that the reaction has ended or at least been significantly slowed down. A decline in the enzyme reaction may, however, not only be due to exhaustion of the substrate. The enzyme stability over time is also a parameter that may affect the reaction. Consequently, if the enzyme is degrading over time this may also cause the reaction to be slowed down. In this case addition of substrate would not result in a renewed decrease in oxygen and pH.

Suitable sources of oxygen include atmospheric air (approx. 20% oxygen), oxygen enriched atmospheric air (oxygen content > 20%) and pure oxygen. Running the process under a pressure higher than 1 atmosphere increases the solubility of oxygen and may be preferred wherever applicable. The oxygen may be supplied to the process, e.g. by continuously mixing air into the reaction mixture during incubation.

An alternative option for providing $O_2$ is by adding $H_2O_2$ in the presence of catalase (see equation 2 above). Alternatively, the $H_2O_2$ naturally produced by the carbohydrate oxidase may by used. Use of $H_2O_2$ as an oxygen source may be particularly preferred when the process is carried out using immobilized enzymes where addition of oxygen is more difficult, or where foam formation, for example in protein containing reaction mixtures, is a problem due to the addition of oxygen by mixing air into the reaction. The catalase may be added at any suitable time e.g. together with the carbohydrate oxidase, or during the reaction, when the $O_2$ level decreases, preferably the catalase is added at incubation start (time = 0). An advantage of adding a catalase together with the carbohydrate oxidase is that the oxygen requirement can be significantly reduced (up to 50%). Thus, supply of oxygen, e.g. in the form of air may be significantly reduced. Actually, by adding an adequate amount of catalase together with $H_2O_2$ it is possible to omit oxygen supplementation completely. This extra-added $H_2O_2$ may originate from any commercial source.

Accordingly, it is preferred that essentially all the oxygen required for the oxidation of maltose to maltobionate is obtained by addition of a catalase, which generates the required oxygen by conversion of the available $H_2O_2$. If the amount of $H_2O_2$ is limiting to the process, additional $H_2O_2$ can be added.

In the present context the expression "essentially all of the oxygen" is used to describe the oxygen supply needed for the enzymatic reaction to work adequately and in particular that it is not necessary to actively add extra oxygen during the process. Preferably, catalase is added in an amount that lowers the concentration of $H_2O_2$ as compared to a similar process without catalase. More preferably, the amount of catalase added to the process as described herein, is an amount that is sufficient to obtain at least 25%, 50%, 75%, 85% or 95% decrease in the amount of $H_2O_2$ as compared to a comparative control process where the only comparative difference is that catalase is not added, even more preferably the amount of catalase added to the process as described herein, is an amount that is sufficient to obtain a 100% decrease in the amount of $H_2O_2$ as compared to a comparative control process where the only comparative difference is that catalase is not added. Preferably, the catalase is added in an amount that also improves the degree of conversion of maltose to maltobionate.

The incubation temperature will generally depend on the carbohydrate oxidase used and is typically selected according to the optimal reaction temperature for the carbohydrate oxidase. However, as the solubility of oxygen decreases with increasing temperature, other factors have to be taken into account in order to obtain an optimal process. The skilled person will know how to balance the optimal temperature with respect to e.g. enzyme activity and oxygen solubility. Generally, a suitable temperature will be in the range from about 0°C to about 99 °C, more preferably in the range of 5°C to 90°C, even more preferably in the range of 15°C to 85°C, most preferably in the range of 25°C to 80°C, even most preferably in the range of 30°C to 60 °C.

The optimal pH can vary dependent on the carbohydrate oxidase used. However, kinetic analysis of carbohydrate oxidase from *Microdochium nivale* (Nordkvist et al., 2007, Biotechnol Bioeng 97: 694-707) indicates that the use of strong bases (NaOH) may affect the stability of carbohydrate oxidases. Furthermore, WO 97/004082 describes that increased yields of lactobionate using carbohydrate oxidase can be obtained when the process is performed at a stable pH. Accordingly, in order to increase the maltobionate yield of the present process it may be desired to maintain pH during the conversion of maltose to maltobionate (step iii, above), by adequate addition of a base, at a stable level. In specific embodiments the stable pH level is maintained in the range of from about 3.0 to about 9.0 by addition of a base. It is possible to maintain pH within the prescribed ranges using any base. In principle, any substance capable of neutralising the produced acid will be applicable in the process. The skilled person knows numerous bases that can be applied in the process of the invention, e.g. strong bases such as $Ca(OH)_2$, KOH, NaOH and $Mg(OH)_2$. In a preferred embodiment a weak base or carbonate is used to maintain the pH at a stable level. Examples of weak bases include, but are not limited to, $CaCO_3$, $Na_2CO_3$, $K_2CO_3$, $(NH_4)_2CO_3$ and $NH_4OH$. Presently, preferred weak bases are $NH_4OH$ and $Na_2CO_3$.

The preferred stable pH value for a specific process of interest will, as it will be appreciated by the skilled person, depend on a number of factors. For instance, if the food product is beer, the production pH of the wort is known to be around 5.0 to 5.7, preferably around 5.1 to 5.3. Thus, it will be preferred to maintain the pH at a stable level around 5.3, such as from pH 5.0 to 5.6. Preferred pH ranges for other food/feed products may be in the range from pH 3.0 to 4.0, e.g. for juice or soft drinks like cola, or in the range from 4.0 to 5.0, e.g. beer or mayonnaise or dressings, or in the range from 5.6 to 6.5, e.g. meat products or in the range from 6.6 to 7.5 milk and egg products.

It will be appreciated that the pH of the maltobionate product or composition comprising maltobionate can also be adjusted to a preferred pH level after or at the end of the enzymatic conversion performed, e.g. when 95% of the desired conversion of maltose has been achieved, the pH may be allowed to drop to a desired level.

In the present context "a stable pH level" is to be broadly understood as the control and maintenance of pH during the process within a specific range, or close to/at a specific value by addition of a base. Control and adjustment/maintenance of pH during an enzymatic process is a standard procedure that can be carried out with a very high degree of accuracy. Thus, a stable pH may be a value maintained at a constant level with a variation of less than 1.5 pH unit, preferably less than 1.0 pH unit, more preferred less than 0.5 pH units, more preferred less than 0.3 pH units, even more preferred less than 0.2 or 0.1 pH units. It follows that an optimal range may be defined for a specific enzymatic process according to the present invention and that pH can be controlled and maintained with the described degree of accuracy within this range. In the process of the invention, a suitable specific pH range or specific pH value is selected in the range from about pH 3 to about pH 9.

It is preferred that pH is maintained at the stable pH level as described herein from the start of the enzymatic reaction. In other words, immediately after the oxidase is added to the maltose containing product the base is added to maintain the stable pH as described herein.

Particularly, if a maximum conversion of maltose is desired the pH is maintained at the stable level as described herein for a period of time that at least last until the oxygen level of the reaction mixture has returned to more than 90% of the initial level, or the amount of base used to keep the pH constant corresponds to the desired degree of conversion.

Preferably, the pH is maintained at the stable pH level as described herein for a time period from 30 minutes to 3 days, preferably, from 2 hours to 48 hours, more preferably from 5 hour to 24 hours, most preferably from 8 hours to 18 hours.

In a particular embodiment of the present invention conversion of maltose to maltobionate is made on a batch of wort obtained from a mashing process. The maltobionate containing wort can be used as ingredient for a series of batches of wort for beer production.

Conversion of starch to maltose to maltobionate is made on a batch of mash prior to the mashing process. The process may include addition of amylase together with carbohydrate oxidase. The maltobionate containing mash can be used as ingredient in a series of mashing processes.

A specific aspect relates to a process for obtaining an increased yield and/or a reduced reaction time in enzymatic conversion of maltose to maltobionate. The process is defined by the steps:

> i) adding a carbohydrate oxidase to a substrate comprising maltose;
> ii) incubating the substrate under conditions allowing the carbohydrate oxidase to convert the maltose to maltobionate; and
> iii) maintaining pH during step ii) in the range of about 3.0 to about 9.0 by addition of a base.

Obviously, the processes disclosed in the present invention are useful for an industrial manufacturing process for production of a beer product where maltose is naturally present during the production.

The present invention relates to the production of maltobionate directly (in situ) in a beer production process by processing maltose which is naturally present in the process. Consequently, the maltobionate is formed during the beer production as such, and not in a separate reaction . The process for producing maltobionate, where the process is integrated into the beer production process comprises the following steps:

> i) adding a oxidoreductases or dehydrogenases, preferably a carbohydrate oxidase, to a beer production process;
> ii) maintaining the process under conditions allowing for the enzymatic conversion of maltose to maltobionate;
> iii) proceeding with the beer production process.

The enzymatic reaction in step i) may be preceded by a starch degrading reaction, for example catalysed by an amylase, which either may be provided endogenously (e.g. from malt already present in the process) or exogenously (e.g. by addition prior to or together with the enzyme of step i). The carbohydrate oxidase and the conditions permitting the enzymatic conversion are essentially the same as described above. With respect to the optimal temperature it should, however, be considered that the reaction is to be run in a beer production process. Consequently, it will be an advantage that the carbohydrate oxidase can perform at the optimal temperatures for such a process.

The advantage of generating maltobionate directly during the beer production process is that once the process has been optimized, separate production steps for the generation of maltobionate are made superfluous.

In the present invention maltobionate is generated during sub-process in a beer production, such as the mashing process or fermentation process, by addition of carbohydrate oxidase and potentially catalase to the sub-process. The conversion of maltose to maltobionate can take place in the mash (grist + fluid) prior to mashing, or during the mashing process or after the cooking of the wort before fermentation, or even during the fermentation. The later, however, requires food approved enzymes. The presence of maltose in wort is, however, needed for the fermentation of the wort into beer. Therefore, the conversion of maltose to maltobionate must be optimized such that the maltose only is converted partially to maltobionate. Preferably, the wort contains up to 2% maltose, more preferred up to 5 % maltose, and most preferred up to 10% maltose.

The mashing process generally applies a controlled stepwise increase in temperature, where each step favors one enzymatic action over the other, eventually degrading proteins, cell walls and starch. Mashing temperature profiles are generally known in the art. In the present invention the conversion of maltose to maltobionate preferably occurs in connection with the saccharification (starch degradation) step between 55°C and 66°C. In a preferred embodiment the carbohydrate oxidase is active in this temperature range. Alternatively, the mashing process may be kept at a lower temperature sufficiently long to allow for the conversion of starch to maltose to maltobionate at a temperature where the carbohydrate oxidase is active. Amylase can be added exogenously at this step to facilitate the conversion of starch to maltose.

**EXAMPLES**

**Example 1**

*Production of maltobionate.*

Na-maltobionate was produced from maltose by oxidation catalysed by a carbohydrate oxidase (M. Nivale CBS 100236 as decribed in WO99/31990) and catalase (Catazyme 25L, Novozymes, Denmark). Enzyme dosages were: carbohydrate oxidase 400 mg enzyme protein/kg maltose and, catalase 6 g/kg maltose. Maltose was dissolved in a concentration of 10%, at 38°C. A stirred reactor containing 3 L solution was used for the reaction. During the reaction atmospheric air was added at 1 L/minute and pH was kept constant at 6.4 by continuous addition of 1 M $Na_2CO_3$ solution. The total reaction time was 17 hours. Essentially all maltose was converted to maltobionic acid during the reaction.

**Example 2**

*Antioxidative effect of maltobionate measured by Ferric Reducing Antioxidant Power (FRAP) assay.*

In brief the FRAP assay functions as follows: $Fe^{3+}$-tripyridyltriazine (TPTZ) complex is reduced to $Fe^{2+}$-TPTZ at low pH. The ferro ($Fe^{2+}$) form is blue coloured and can be measured spectro-photometrically at 593 nm. The method is calibrated with known concentrations of Fe2+ solutions. The higher the absorbance means higher antioxidative status.
For the FRAP assay a working reagent was prepared from the following components:

Acetatebuffer: 3.1 g CH3COONa·3H20 and 16 mL conc. CH3COOH in ≈ 800mL water.

Check that the pH is 3.6. Otherwise adjust with NaOH/CH3COOH. Add water to 1 L.

TPTZ solution: 10mmol/L 2,4,6-Tri(2-pyridyl)-s-triazine (TPTZ) in 40mmol/L HCl

Fe(III) solution: 20 mM Fe(III)CL$_3$·6H2O.

Working reagent (prepared daily): 50 mL acetatebuffer + 5.0 mL TPTZ solution + 5.0 mL Fe(III) solution
The assay was performed by adding 50 μL sample/standard/blank to 1.5 mL working reagent in 2 mL dark eppendorf vials followed by incubation in thermo mixer at 37°C for 30 min. Samples consist of maltobionate in different concentrations, standards contain Fe2+ and ascorbic acid, and blanks were water samples. The assay was performed in triplicate. The absorbance was read immediately at 593 nm, the higher the absorbance the higher antioxidative status (↑Abs →), Antioxidant ↑). The results are presented in Table 1.

Table 1: Sodium maltobionate in FRAP (g/L) Abs at 593 nm

| Sodium maltobionate (liquid concentrates) | | |
|---|---|---|
| | Abs Average | Std. Dev. |
| 5% | 0.236 | 0.021 |
| 2,50% | 0.170 | 0.002 |
| 1% | 0.112 | 0.006 |
| Blank | 0.0257 | 0.00058 |

Maltobionate show anti-oxidative capacity in terms of reducing Fe(III) and thus creating a change in absorbance due to the ferrous-tripyridyltriazine complex. The assay showed a clear dose response effect of the antioxidant.

**Example 3**

*Antioxidative effect of carbohydrate oxidase and/or catalase in beer wort.*

Beer wort was produced from well modified barley malt 50g in 250g water at 53 °C. The barley malt was mashed using a temperature profile consisting of 30 min at 52 °C, increase by 1°C/min for 11 min, 63 °C for 30 min, increase by 1 °C/min for 9 min, 72°C for 30 min, increase by 1 °C/min for 6 min, 78°C for 15 min, followed by cooling to 20°C.

A series of experiments with addition of carbohydrate oxidase and/or catalase to the wort were made and compared to a control sample. The enzymes were added prior to the mashing step. Carbohydrate oxidase was dosed according to the activity measured as LOXU where one LOXU corresponds to 1 mg enzyme protein. Catalase was dosed according to the activity measured as CIU. 1 CIU is the amount of enzyme that decompose 1 $\mu$mol $H_2O_2$ per minute at pH = 7.0 and T= 25 °C

To measure the antioxidant capacity of the carbohydrate oxidase and/or catalase, 1mM $Fe^{2+}$ was added to all samples. Oxidation was measured in0064irectly using a Xylenol orange complex assay (XO-assay). In this assay hydroperoxides in the wort oxidizes $Fe^{2+}$ in the xylenol orange-complex to $Fe^{3+}$ which forms a coloured complex with xylenol orange. The oxidized complex can be measured spectrophotometrically, the lower the absorbance the higher antioxidative status ($\downarrow$abs $\rightarrow$ Antioxidant $\uparrow$).

The XO working reagent was prepared from the following components:

A: 2.5 mM Ammonium Ferrous sulfate hexahydrate, 1.0 mM Xylenol orange tetrasodium salt (XO) in 1250mM $H_2SO_4$

B: 4.89 mM Butylated Hydroxytoluene (BHT) in methanol.

XO working reagent: Mix 1 part A with 9 parts B. Stable 1 month in fridge if kept in dark bottle.

The assay was initiated by adding 100 $\mu$L sample to 900 $\mu$L XO working reagent. Incubation was performed at room temperature for 30 min with stirring. Each sample was centrifuged at 14000 rpm at 20°C for 10 minutes. Absorption of the supernatant was measured spectrophotometrically at 560nm.

Absorbance increased in all samples due to increase in coloured XO complex with oxidized Fe2+ to Fe3+. The wort samples with 50 LOXU prevents formation of oxidation compared to the control(abs = 0.410 control and 0.257 for the 50LOXU). The effect is poor in half the dosage 25 LOXU (abs = 0.421), however improved by combination of 600 CIU Catalase (abs = 0.308). 1200 CIU of Catalase also prevents formation of coloured complex (abs = 0.264) and the effect is dosage depended as 600 CIU is hardly different (abs = 0.439) from the control.

**Claims**

1. A process for producing maltobionate comprising the following steps:

    a) obtaining a maltose containing substrate applicable in a wort or mash production process; and
    b) converting the maltose to maltobionate by an enzymatic reaction, wherein the maltobionate is formed during a sub-process in a beer production.

2. The process according to claim 1, where the enzymatic reaction in step b) of claim 1 is catalyzed by an oxidoreductase.

3. The process according to claim 2, where the oxidoreductase is a carbohydrate oxidase.

4. The process according to claim 3, where the carbohydrate oxidase is selected from hexose oxidase, cellobiose oxidase, aldose oxidase and pyranose oxidase.

5. The process according to claim 3, where the carbohydrate oxidase is obtainable from *Microdochium nivale* deposited under CBS 100236.

6. The process according to any one of the preceding claims, where a catalase is added to the process.

7. The process according to claim 6, where the catalase is added to the enzymatic reaction of step b) of claim 1.

8. The process according to claim 1, wherein the sub-process is the mashing process.

9. The process according to any one of the preceding claims, where step b) of claim 1 is maintained at a stable pH level by addition of a base during the process.

**Patentansprüche**

1. Verfahren zum Herstellen von Maltobionat, umfassend die folgenden Schritte:

   a) Erhalten eines Maltose enthaltenden Substrats, das in einem Stammwürze-oder Maischeherstellungsverfahren einsetzbar ist; und
   b) Umsetzen der Maltose durch eine enzymatische Reaktion zu Maltobionat, wobei das Maltobionat während eines Unterverfahrens einer Bierherstellung gebildet wird.

2. Verfahren nach Anspruch 1, wobei die enzymatische Reaktion in Schritt b) von Anspruch 1 durch eine Oxidoreduktase katalysiert wird.

3. Verfahren nach Anspruch 2, wobei die Oxidoreduktase eine Kohlenhydratoxidase ist.

4. Verfahren nach Anspruch 3, wobei die Kohlenhydratoxidase ausgewählt ist aus Hexoseoxidase, Cellobioseoxidase, Aldoseoxidase und Pyranoseoxidase.

5. Verfahren nach Anspruch 3, wobei die Kohlenhydratoxidase aus *Microdochium nivale* erhältlich ist, hinterlegt unter CBS 100236.

6. Verfahren nach einem beliebigen der vorhergehenden Ansprüche, wobei eine Katalase zu dem Verfahren hinzugefügt wird.

7. Verfahren nach Anspruch 6, wobei die Katalase zu der enzymatischen Reaktion von Schritt b) in Anspruch 1 hinzugefügt wird.

8. Verfahren nach Anspruch 1, wobei das Unterverfahren das Maischeverfahren ist.

9. Verfahren nach einem beliebigen der vorhergehenden Ansprüche, wobei Schritt b) von Anspruch 1 bei einem stabilen pH-Level durch Hinzufügen einer Base während des Verfahrens gehalten wird.


**Revendications**

1. Procédé de production de maltobionate comprenant les étapes suivantes :

   a) obtenir un maltose contenant du substrat applicable à un procédé de production de moût ou de broyat ; et
   b) transformer le maltose en maltobionate par une réaction enzymatique, dans lequel le maltobionate est formé pendant un sous-procédé de production de bière.

2. Procédé selon la revendication 1, où la réaction enzymatique de l'étape b) de la revendication 1 est catalysée par une oxydoréductase.

3. Procédé selon la revendication 2, où l' oxydoréductase est une carbohydrate oxydase.

4. Procédé selon la revendication 3, où la carbohydrate oxydase est sélectionnée parmi hexose oxydase, cellobiose oxydase, aldose oxydase et pyranose oxidase.

5. Procédé selon la revendication 3, où la carbohydrate oxydase peut être obtenue à partir de *Microdochium nivale* déposée sous le numéro CBS 100236.

6. Procédé selon l'une quelconque des revendications précédentes, où une catalase est ajoutée au procédé.

7. Procédé selon la revendication 6, où la catalase est ajoutée à la réaction enzymatique de l'étape b) de la revendication 1.

8. Procédé selon la revendication 1, dans lequel le sous-procédé est le procédé de broyage.

**9.** Procédé selon l'une quelconque des revendications précédentes, où l'étape b) de la revendication 1 est maintenue à un niveau de pH stable par addition d'une base pendant le procédé.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3899604 A
- US 3829583 A
- EP 0384534 B1
- WO 9640935 A
- JP 5084074 B
- WO 9931990 A
- US 2496297 A
- US 3862005 A
- EP 384534 A
- WO 97004082 A

**Non-patent literature cited in the description**

- *App Env Microbiol,* 2005, 7846-7857
- **BEAN ; HASSID.** *J Biol Chem,* 1956, vol. 218, 425-436
- **SCHOU et al.** *Biochemical Journal,* 1998, vol. 330, 565-571
- **LIN et al.** *Biochimica et Biophysica Acta,* 1991, vol. 1118, 41-47
- **NORDKVIST et al.** *Biotechnol Bioeng,* 2007, vol. 97, 694-707